# EUROPEAN PATENT SPECIFICATION

(11) **EP 1 838 258 B1**
(45) Date of publication and mention of the grant of the patent: **08.12.2010**
(21) Application number: 05792289.0
(22) Date of filing: 11.10.2005
(51) Int. Cl.: A61F 5/451, A61F 13/537, A61G 9/00, A61L 15/60

(54) **A DEVICE FOR COLLECTING URINE OR OTHER ORGANIC BODY FLUIDS, A LAMINATE SHEET AND A METHOD FOR PRODUCING SAID SHEET**
VORRICHTUNG ZUM SAMMELN VON URIN ODER ANDEREN ORGANISCHEN KÖRPERFLÜSSIGKEITEN, LAMINATFOLIE UND VERFAHREN ZUR HERSTELLUNG DER FOLIE
DISPOSITIF POUR RECUEILLIR DE L'URINE OU D'AUTRES FLUIDES CORPORELS ORGANIQUES, LAMINE ET PROCEDE POUR PRODUIRE LE LAMINE

(30) Priority: 12.10.2004 SE 0402468
(43) Date of publication of application: 03.10.2007
(73) Proprietor: Brodén, Bengt-Inge, 532 38 Skara (SE)
(72) Inventor: Brodén, Bengt-Inge, 532 38 Skara (SE)
(74) Representative: Fritzon, Rolf
(86) International application number: PCT/SE2005/001505
(87) International publication number: WO 2006/041393

(56) References cited:
- EP-A- 1 177 781
- EP-A2- 1 177 781
- WO-A-02/49551
- GB-A- 232 429
- GB-A- 2 301 350
- GB-A- 2 301 350
- US-A- 5 007 116
- US-B1- 6 186 990
- US-B1- 6 659 986

## Description

### TECHNICAL FIELD

The present invention relates to a device for collecting urine or other organic body fluids, comprising a flexible bag made of liquid-impermeable film material, a member for receiving fluid and conveying it into the bag, means for preventing fluid from leaving the bag via said receiving member, and at least one absorption body arranged in the bag and in the form of a laminate sheet comprising a superabsorbent material which is enclosed between two liquid-permeable surface layers and which forms a gel composition when delivered fluid is absorbed.

The invention also relates to a laminate sheet included in the device, and to a method for producing such a laminate sheet.

### BACKGROUND TO THE INVENTION

A device of the kind specified in the introduction eliminates, among other things, many of the problems associated with the previous use of bottles and bedpans by patients confined to wheelchairs and patients who are bedridden. Furthermore, the device improves hygiene both for patients and nursing personnel, and much of the unpleasantness involved in handling bedpans can be eliminated, because the urine is handled while enclosed in liquid-impermeable plastic bags that are intended to be discarded.

Collection devices of this kind can also be used by healthy people in many circumstances, for example in automotive vehicles, boats and aircraft, and by persons who spend long periods of time in other enclosed spaces or are not able to leave a position, for example when on guard duty.

A device of the type mentioned above, but without superabsorbent material, is described in my European patent EP 0 772 430.

To avoid handling bags filled with fluid, such as urine, it has previously been proposed to place a superabsorbent material in collection bags. When urine, for example, is delivered to such a bag, the superabsorbent material, upon absorption of the delivered urine, will be converted to a gel composition, which has several advantages in terms of the use of the bag and its subsequent handling.

US patent specification No. 6186990 describes a urine-collecting bag which comprises a superabsorbent material enclosed in a liquid-permeable envelope. All the superabsorbent material in the liquid-permeable envelope is exposed in its entirety upon each delivery of new urine to the bag. This means, for example, that the superabsorbent material in the urine-collecting bag will work effectively when urine in normal amounts is delivered a first time and perhaps a second time, whereas upon further delivery of urine a liquid mixture is obtained which is unable to bind the newly delivered urine.

GB 2301350 A describes a method for producing a superabsorbent product which can have the shape of a laminate sheet comprising two surface layers and a superabsorbent material bound between these.

EP 1177781 A2 describes the use of a superabsorbent body in the form of a laminate sheet which is said to increase the amount of urine that can be absorbed and bound in the form of a gel in a urine-collecting bag. The preamble of appended claim 1 is based on this document.

According to the last-mentioned patent specification, the bag is designed with at least two chambers, the urine being delivered to a first receiving chamber from which it flows across to a second collecting chamber in which the superabsorbent laminate sheet is arranged. To prevent urine from coming into contact with the upper part of the laminate sheet, the upper part of the collecting chamber is separated from the receiving chamber by a liquid-impermeable wall. Moreover, the laminate sheet is intended to be arranged on, for example bonded to, a wall of the bag so that it is at all times upright and extends over the entire height of the collecting chamber. This known arrangement is relatively complicated to produce since it requires a number of extra production stages for forming an intermediate wall with a liquid-impermeable part and a liquid-permeable part, and requires the arrangement of the laminate sheet such that the latter is constantly maintained upright.

International patent application WO 02/49551 discloses a disposable excreta management device comprising a superabsorbent material coverable by a liquid-permeable topsheet.

### OBJECT OF THE INVENTION

It is an object of the present invention to provide a urine-collecting device of the kind mentioned in the first paragraph, which device, despite comprising an effective superabsorbent material, is simple and inexpensive to produce and easy to use and can be handled hygienically after use. A laminate sheet according to the invention is defined in independent claim 1.

Another object is to make available a simple and cost-effective method of producing a superabsorbent laminate sheet included in a device according to the invention, as defined in independent claim 11.

The invention is based on the recognition that the first of the abovementioned objects can be achieved by designing the laminate sheet such that its surface layers themselves prevent delivered fluid in brief contact with the surface layer from penetrating through the latter, but fluid in contact with the surface layer is rapidly conveyed through this. Such a laminate sheet can be placed loosely in a urine-collecting bag without the need for any intermediate wall protecting the upper part of the laminate sheet from contact with the delivered urine.

To ensure that the correct amount of superabsorbent material is always used for absorption of delivered urine for converting the latter to a gel composition, the laminate sheet according to the invention must be allowed to collapse or "break up" from its lower end upward at the rate at which the urine to be converted to gel is delivered. Thus, no more superabsorbent material is used than is actually necessary on each occasion for conversion of the actual amount of urine, and this means an optimal utilization of the available superabsorbent material in the bag.

The special characteristic of a device of the type mentioned in the first paragraph is that the surface layers of the laminate sheet have such a porosity that delivered fluid in brief contact with a surface layer of a non-horizontal laminate sheet will run down the outside of the latter without penetrating to any appreciable extent through the surface layer, whereas delivered fluid which forms a fluid collection in contact with a surface layer of the laminate sheet penetrates through the latter so as to be absorbed by the superabsorbent material located to the inside of the surface layer and so as to convert this superabsorbent material to a gel composition.

In a preferred embodiment of a device according to the invention, the laminate sheet is intended to be supported by the bottom of the bag, the surface layers of the laminate sheet being made of a flexible material which allows the laminate sheet to collapse toward the bottom of the bag as the superabsorbent material in the laminate sheet is converted to a gel composition by the delivered fluid.

The surface layers of the laminate sheet are preferably made of compressed tissue material. This material has such a pore size that fluid in brief contact with a surface layer does not pass through the latter.

According to the invention, the superabsorbent laminate sheets are produced by two layers of tissue material and one intermediate layer of superabsorbent material being rolled together to form a blank from which laminate sheets of the desired size are punched out. A roll pressure is used which gives the blank such stiffness that the laminate sheets punched out from it are self-supporting and can be placed on the bottom of a collection bag intended for receiving urine or other organic body fluids, and which roll pressure compresses the tissue material in the surface layers of the laminate sheets in order to reduce the pore size of these to such a degree that fluid in brief contact with a surface layer does not penetrate to any appreciable extent through the latter.

The tissue material in the surface layers of the laminate sheets is expediently compressed to a density of 0.7 g/cm³ ± 20%, preferably 0.7 g/cm³ ± 10%, and most preferably 0.7 g/cm³ ± 5%.

On application of the above-described process, the laminate sheet can be produced such that the tissue material in the surface layers constitutes 2-6 percent by weight, preferably ca. 3 percent by weight of the laminate sheet.

It is preferable that the surface layers of tissue material are compressed to such a degree that they acquire a pore size of 200 µm ± 100 µm, preferably 250 µm ± 50 µm.

To ensure that loose superabsorbent material in the laminate sheet does not become detached and settle on areas of the collection bag which are to be welded together during the latter's production, something which may lead to weld seams that are not completely leaktight, it is preferable that at least the side edges of the punched-out laminate sheets are moistened so that the superabsorbent material located between the edge portions of the surface layers is converted to a gel strand which closes the side edges. The moistening is expediently done with the aid of water vapor.

In another preferred embodiment, the superabsorbent material in the laminate sheet is divided into mutually separate sections, from the lower end portion of the laminate sheet upward. This can be done by the surface layers being joined and bonded to one another along transverse connection lines. The result of this is that fluid delivered to the bag converts the superabsorbent material to a gel composition in one section after another, starting in the section located at the bottom of the bag. Thus, superabsorbent material is used up in only so many sections as are necessary on each occasion, which means optimal utilization of the superabsorbent material.

Other features of the invention will become apparent from the patent claims.

The invention will be described in more detail below with reference to the illustrative embodiments shown in the accompanying drawings.

### BRIEF DESCRIPTION OF THE FIGURES

Fig. 1 is a perspective view of a device according to the invention.
Fig. 2 illustrates a device according to the invention after it has received a first quantity of urine.
Fig. 3 is a perspective view of an embodiment of a superabsorbent laminate sheet which can be included in the device according to Figures 1 and 2.
Fig. 4 is an enlarged detail through an edge portion of the laminate sheet in Fig. 3.
Fig. 5 illustrates a second embodiment of a device according to the invention.

### DETAILED DESCRIPTION OF THE PREFERRED EMBODIMENTS

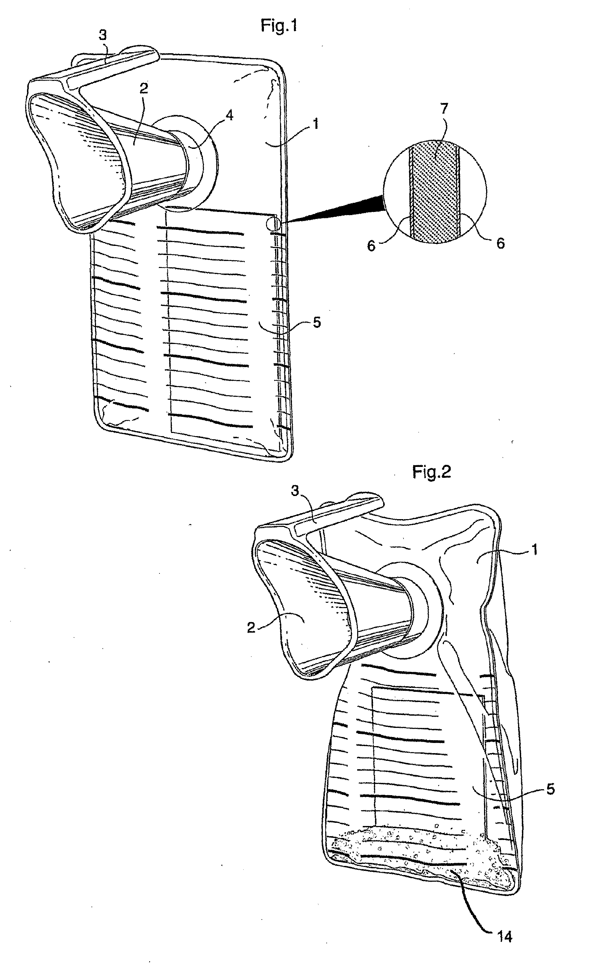

The collecting device according to Fig. 1 comprises a liquid-impermeable plastic film which has been folded and welded together to form a bag 1. A funnel-shaped receiving member 2, intended to receive urine and convey it into the bag, is fitted near one short side of the substantially rectangular bag 1. This facilitates use of the bag, even by a bed-ridden patient. The upper contour of the funnel is precisely shaped to allow the device to be placed in a leaktight manner against the skin when used by women.

To make it easier to press the funnel against the skin, it is provided with a handle 3. To fit the funnel on the bag 1, the bag is provided with a mounting flange 4 in which the funnel 2, by virtue of its conical shape, can be pressed down to provide a liquid-tight abutment against the flange 4. Two substantially semicircular cuts (not shown) are formed within the area of the upper film sheet of the bag 1 enclosed by the flange 4. These cuts form two flaps which can cooperate with a bottom surface of the funnel to produce a nonreturn valve function. This is described in more detail in my abovementioned European patent EP 0 772 430. The funnel with the bag can be designed entirely in the manner described in said patent, which is incorporated by reference in the present application. The fitting of the funnel on the bag and said nonreturn valve function will not be described in any more detail here.

Reference number 5 designates a superabsorbent laminate sheet arranged in the bag 1. This laminate sheet comprises (see enlarged section in Fig. 1) two surface layers 6 and an intermediate layer 7 of superabsorbent material, expediently in powder form with a particle size of less than 600 µm. The superabsorbent material used can be crosslinked sodium polyacrylate or another material having superabsorbent properties, for example one of those specified in GB 2325432 A. The important point is that the superabsorbent material must have extremely high absorbency and, upon absorption of fluid such as urine, must be converted rapidly to a gel composition.

The surface layers 6 are made of tissue material produced from pure bleached cellulose which is compressed to give very thin, flexible, gauze-like layers with no inherent stiffness. The superabsorbent laminate sheet is produced by two or more layers of tissue material and one intermediate layer of superabsorbent material being rolled together to form a blank from which laminate sheets of the desired size are punched out. This rolling-together can in principle be carried out in the manner illustrated in GB 2301350 A.

According to the present invention, a roll pressure is used which gives the blank such stiffness that the laminate sheets punched out from it are self-supporting and can be placed on the bottom of a collection bag intended to receive urine or other organic body fluids. The roll pressure is also chosen such that the tissue material in the surface layers of the laminate sheets is compressed to such an extent that the desired porosity thereof is achieved. The desired porosity is determined by which fluid is to be absorbed by the laminate sheet. The porosity must be such that the fluid in question, in brief contact with a surface layer of a non-horizontal laminate sheet, will run down the outside of the latter without penetrating to any appreciable extent through the surface layer, whereas delivered fluid which forms a fluid collection in contact with a surface layer of the laminate sheet penetrates through this so as to be absorbed by the superabsorbent material located to the inside of the surface layer and so as to convert this superabsorbent material to a gel composition. The appropriate rolling pressure will depend on the number and nature of the tissue material layers and can be determined by the skilled person through tests using several different roll pressures and through checks of how the obtained laminate sheet reacts with the intended fluid.

For a superabsorbent laminate sheet intended to be used in a bag for collecting urine, it has been found expedient to compress the tissue material to such an extent that the pore size obtained is ca. 200 µm ± 100 µm, preferably 250 µm ± 50 µm. In a laminate sheet with surface layers having such a pore size, no appreciable amount of the urine running along the laminate sheet will penetrate through the surface layer. By contrast, urine in a collection of fluid surrounding such a laminate sheet will rapidly penetrate through the surface layers and be absorbed by the superabsorbent material located to the inside of these. The amount of superabsorbent material in a laminate sheet can be 30 g ± 5 g, preferably 28 g ± 3 g.

The above-described compression of the tissue material in the surface layers of the laminate sheets can be expediently adapted so that these acquire a density of 0.7 g/cm³ ± 20%, generally 0.7 g/cm³ ± 10%. The tissue material can constitute 2-6 percent by weight, preferably ca. 3 percent by weight of the laminate sheet, whose thickness can be 2 mm.

Fig. 2 shows the device according to Fig. 1 after the bag 1 has received a quantity of urine corresponding to a normal discharge of urine. The urine has been delivered to the bag 1 via the funnel 2 and has run down along the surface layers of the superabsorbent laminate sheet 5. Because the porosity of the surface layers has been chosen in accordance with the above, no appreciable amount of the delivered urine has passed through the surface layers. The delivered urine has gathered at the bottom of the bag and has formed a fluid collection around the lower part of the laminate sheet. The urine in this fluid collection has been able to pass rapidly through the pores in the surface layers of the laminate sheet and has converted the intermediate superabsorbent material to a gel composition 14. The lower part of the laminate sheet 5 has collapsed toward the bottom of the bag 1 or been broken up. In accordance with the above, the surface layers 6 have no inherent bearing capacity when the superabsorbent material has been converted to a gel together with the urine.

The part of the laminate sheet 5 whose superabsorbent material has not been needed for the absorption of the delivered amount of urine and for the gel formation is still substantially intact in the bag 1 above the gel composition 14. When the next quantity of urine is delivered in conjunction with the next discharge, another part of the laminate sheet 5 is used, and so on until the entire laminate sheet is broken up. By virtue of the fact that the laminate sheet 5 is used only successively for the gel formation, a device according to the invention affords a very high overall capacity when the superabsorbent material is used optimally. This is also important from the point of view of cost.

When the whole laminate sheet 5 has been used up and the bag is substantially filled with a gel composition, the whole bag can be disposed of. By virtue of the nonreturn valve, this can be done without any risk of leakage. Moreover, it is more pleasant for the user if, between passing urine, he or she can keep a bag filled partially with gel rather than a bag which is partially filled with fluid.

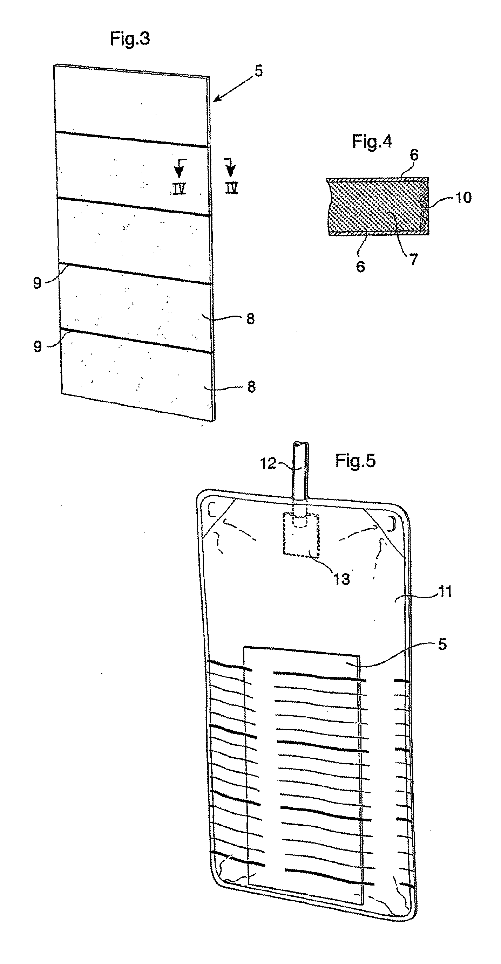

Figures 3 and 4 show a further development of a superabsorbent laminate sheet 5 according to the present invention. In this embodiment, the laminate sheet 5 is divided into a number of sections 8 from the lower end portion of the laminate sheet upward. Each section 8 contains a part of the superabsorbent material included in the laminate sheet 5, the material in one section being kept apart from the material in adjoining sections. This means that fluid delivered to a bag in which the laminate sheet is arranged converts the superabsorbent material in the laminate sheet to a gel composition in one section after another, starting in the section located at the bottom of the bag. This further ensures that no more superabsorbent material is used than is actually needed on each occasion. The sections 8 can be separated from one another by the surface layers having been joined and bonded to one another along transverse connection lines 9.

As has been described above, the laminate sheets 5 are punched out from a relatively stiff blank. There is a risk here of particles from the superabsorbent layer 7 between the surface layers 6 coming loose. These particles can then settle on areas of the collection bag which are to be welded together during its production. If this happens, it may result in weld seams that leak. It is therefore preferred that the side edges of each laminate sheet 5 are sealed together.

According to the present invention, this can be done by moistening the side edges of the laminate sheet 5, for example with the aid of vapor, so that the outermost superabsorbent material 7 located between the surface layers 6 is converted to a gel strand 10 (see Fig. 4) which closes the edge portions. This closure can also contribute to preventing urine that runs down along the surface layers 6 of the laminate sheet 5 from penetrating into the superabsorbent material between these on its way down to the lower portion of the laminate sheet.

Fig. 5 illustrates another embodiment of a device according to the invention comprising a catheter bag 11 intended to collect urine delivered to the bag via a line 12. Reference number 13 designates a simple nonreturn valve mechanism consisting of two film portions bearing against one another. A superabsorbent laminate sheet 5 of the previously described configuration is arranged in the catheter bag 11.

A catheter bag of this kind can, for example, be secured on a leg. For the user, it is a great advantage that urine delivered to the bag is converted directly to a gel composition with the aid of the superabsorbent laminate sheet 5 arranged in the bag in a manner corresponding to that described above.

The invention has been described above in connection with the embodiments shown in the drawings. However, a device according to the invention can be varied in several respects within the scope of the patent claims, among other things for adaptation to other applications in which body fluid is to be collected. For example, any desired material for the surface layers can be chosen as long as it has the characteristic specified in the patent claims. The superabsorbent laminate sheets can be produced not just by rolling, but also by pressing.

## Claims

1. A laminate sheet (5) for arrangement in a bag of a device for collecting urine or other organic body fluids, comprising a superabsorbent material (7) which is enclosed between two liquid-permeable surface layers (6) and which forms a gel composition (14) when delivered fluid is absorbed, **characterized in that** the surface layers (6) of the laminate sheet (5) have a specifically adapted pore size such that delivered fluid in brief contact with a surface layer of the non-horizontal laminate sheet (5) will run down the outside of the latter without penetrating to any appreciable extent through the surface layer (6), whereas delivered fluid which forms a fluid collection in contact with a surface layer of the laminate sheet penetrates through the latter so as to be absorbed by the superabsorbent material (7) located to the inside of the surface layer and so as to convert this superabsorbent material (7) to a gel composition (14).

2. The laminate sheet as claimed in claim 1, **characterized in that** it contains 30 g ± 5 g, preferably 28 g ± 3 g of superabsorbent material (7).

3. A device for collecting urine or other organic body fluids, comprising a flexible bag (1; 11) made of liquid-impermeable film material, a member (2; 12) for receiving fluid and conveying it into the bag, means (13) for preventing fluid from leaving the bag via said receiving member, and at least one absorption body arranged in the bag (1), **characterized in that** said at least one absorption body is a laminate sheet (5) as claimed in claim 1 or 2.

4. The device as claimed in claim 3, **characterized in that** the laminate sheet (5) is supported by the bottom of the bag (1), **in that** the surface layers (6) of the laminate sheet are made of a flexible material which allows the laminate sheet to collapse toward the bottom of the bag as the superabsorbent material (7) in the laminate sheet is converted to a gel composition (14) by delivered fluid.

5. The device as claimed in claim 3 or 4, **characterized in that** the surface layers (6) of the laminate sheet (1) are made of compressed tissue material.

6. The device as claimed in claim 5, **characterized in that** the tissue material in the surface layers (6) is compressed to a density of 0.7 g/cm³ ± 20%, preferably 0.7 g/cm³ ± 10%.

7. The device as claimed in any of claims 5-6, **characterized in that** the tissue material constitutes 2-6 percent by weight, preferably ca. 3 percent by weight of the laminate sheet.

8. The device as claimed in any of claims 5-7, **characterized in that** the tissue material has a pore size of 200 µm ± 100 µm, preferably 250 µm ± 50 µm.

9. The device as claimed in any of claims 3-8, **characterized in that** the edge portions of the laminate sheet (5) are closed with a gel strand (10) consisting of superabsorbent material (7) converted to gel and arranged between the edge portions of the surface layers (6).

10. The device as claimed in any of claims 3-9, **characterized in that** the laminate sheet (5) is divided into sections (8) from the lower end portion of the laminate sheet and upward, **in that** each section (8) contains part of the superabsorbent material (7) included in the laminate sheet, **in that** the material in one section is kept separate from the material in adjoining sections so that fluid delivered to the bag (1; 11) converts the superabsorbent material (7), in the laminate sheet (5) supported by the bottom of the bag, to a gel composition (14) one section after another, starting in the section located at the bottom of the bag.

11. A method for producing superabsorbent laminate sheets (5) as claimed in claim 1 or 2, in which two layers of tissue material (6) and an intermediate layer of superabsorbent material (7) are rolled together to form a blank from which laminate sheets of the desired size are punched out, **characterized in that** a roll pressure is used which gives the blank such stiffness that the laminate sheets punched out from it are self-supporting and can be placed on the bottom of a collection bag (1,11) intended for receiving urine or other organic body fluids, and which roll pressure compresses the tissue material in the surface layers of the laminate sheets in order to reduce the pore size of these to such a degree that fluid in brief contact with a surface layer of a non-horizontal laminate sheet will run down the outside of the latter without penetrating to any appreciable extent through the surface layer, whereas delivered fluid which forms a fluid collection in contact with a surface layer of the laminate sheet penetrates through the latter so as to be absorbed by the superabsorbent material located to the inside of the surface layer and so as to convert this superabsorbent material to a gel composition (14).

12. The method as claimed in claim 11, **characterized in that** the tissue material in the surface layers of the laminate sheets is compressed to a density of 0.7 g/cm³ ± 20%, preferably 0.7 g/cm³ ± 10%.

13. The method as claimed in claim 11 or 12, **characterized in that** the tissue material is compressed to such an extent that it acquires a pore size of 200 µm ± 100 µm, preferably 250 µm ± 50 µm.

14. The method as claimed in one of claims 11-13, **characterized in that** at least the side edges of the punched-out laminate sheets are moistened so that the superabsorbent material located between the edge portions of the surface layers is converted to a gel composition (10) closing the side edges of the laminate sheets.

15. The method as claimed in claim 14, **characterized in that** said side edges are moistened with the aid of water vapor.

16. The method as claimed in any of claims 11-15, **characterized in that** the superabsorbent material in the laminate sheet is divided into mutually separate sections (8), from the lower end portion of the laminate sheet upward.

17. The method as claimed in claim 16, **characterized in that** the division of the laminate sheet is effected by the surface layers being joined and bonded to one another along transverse connection lines (9).

## Patentansprüche

1. Laminatfolie (5) zum Anordnen in einem Beutel einer Vorrichtung zum Sammeln von Urin oder anderen organischen Körperflüssigkeiten, umfassend ein stark absorbierendes Material (7), das zwischen zwei flüssigkeitsdurchlässigen Flächenschichten (6) eingeschlossen ist, und das eine Gelzusammensetzung (14) bildet, wenn abgegebene Flüssigkeit absorbiert wird, **dadurch gekennzeichnet, dass** die Flächenschichten (6) der Laminatfolie (5) eine spezifisch angepasste Porengröße aufweisen, so dass abgegebene Flüssigkeit, die in kurzem Kontakt mit einer Flächenschicht der nicht horizontalen Laminatfolie (5) steht, außen an Letzterer herabfließt, ohne in nennenswertem Maße die Flächenschicht (6) zu durchdringen, wobei abgegebene Flüssigkeit, die eine Flüssigkeitssammlung bildet, die in Kontakt mit einer Flächenschicht der Laminatfolie steht, Letztere durchdringt, so dass sie von dem stark absorbierenden Material (7) absorbiert wird, das sich innen an der Flächenschicht befindet, und so dass dieses stark absorbierende Material (7) in eine Gelzusammensetzung (14) umgewandelt wird.

2. Laminatfolie nach Anspruch 1, **dadurch gekennzeichnet, dass** sie 30 g ± 5 g, vorzugsweise 28 g ± 3 g stark absorbierenden Materials (7) umfasst.

3. Vorrichtung zum Sammeln von Urin oder anderen organischen Körperflüssigkeiten, umfassend einen flexiblen Beutel (1; 11), hergestellt aus flüssigkeitsundurchlässigem Filmmaterial, ein Element (2; 12) zum Aufnehmen von Flüssigkeit und Bringen der Flüssigkeit in den Beutel, ein Mittel (13), um zu verhindern, dass Flüssigkeit über das Aufnahmeelement den Beutel verlässt, und mindestens einen Absorptionskörper, der in dem Beutel (1) angeordnet ist, **dadurch gekennzeichnet, dass** der mindestens eine Absorptionskörper eine Laminatfolie (5) nach Anspruch 1 oder 2 ist.

4. Vorrichtung nach Anspruch 3, **dadurch gekennzeichnet, dass** die Laminatfolie (5) von dem Unterteil des Beutels (1) **dadurch** getragen wird, dass die Flächenschichten (6) der Laminatfolie aus einem flexiblen Material hergestellt sind, das ermöglicht, dass die Laminatfolie zum Unterteil des Beutels hin zusammenfällt, wenn das stark absorbierende Material (7) in der Laminatfolie durch abgegebene Flüssigkeit in eine Gelzusammensetzung (14) umgewandelt wird.

5. Vorrichtung nach Anspruch 3 oder 4, **dadurch gekennzeichnet, dass** die Flächenschichten (6) der Laminatfolie (1) aus gepresstem Gewebematerial bestehen.

6. Vorrichtung nach Anspruch 5, **dadurch gekennzeichnet, dass** das Gewebematerial in den Flächenschichten (6) auf eine Dichte von 0,7 g / cm³ ± 20 %, vorzugsweise 0,7 g / cm³ ± 10 %, gepresst ist.

7. Vorrichtung nach einem der Ansprüche 5 - 6, **dadurch gekennzeichnet, dass** das Gewebematerial 2 - 6 Gewichtsprozent, vorzugsweise ca. 3 Gewichtsprozent der Laminatfolie darstellt.

8. Vorrichtung nach einem der Ansprüche 5 - 7, **dadurch gekennzeichnet, dass** das Gewebematerial eine Porengröße von 200 µm ± 100 µm, vorzugsweise 250 µm ± 50 µm, aufweist.

9. Vorrichtung nach einem der Ansprüche 3 - 8, **dadurch gekennzeichnet, dass** die Kantenabschnitte der Laminatfolie (5) mit einem Gelstrang (10) geschlossen sind, der aus einem stark absorbierenden Material (7) besteht, das in Gel umgewandelt und zwischen den Kantenabschnitten der Flächenschichten (6) angeordnet ist.

10. Vorrichtung nach einem der Ansprüche 3 - 9, **dadurch gekennzeichnet, dass** die Laminatfolie (5) von dem unteren Endabschnitt der Laminatfolie und aufwärts in Abschnitte (8) geteilt ist, dass jeder Abschnitt (8) einen Teil des stark absorbierenden Materials (7), das von der Laminatfolie umfasst ist, umfasst, dass das Material in einem Abschnitt getrennt von dem Material in benachbarten Abschnitten gehalten wird, so dass Flüssigkeit, die in den Beutel (1; 11) abgegeben wird, abschnittsweise, beginnend in dem Abschnitt, der sich am Unterteil des Beutels befindet, das stark absorbierende Material (7) in der Laminatfolie (5), die von dem Unterteil des Beutels getragen wird, in eine Gelzusammensetzung (14) umwandelt.

11. Verfahren zum Herstellen stark absorbierender Laminatfolien (5) nach Anspruch 1 oder 2, in dem zwei Schichten aus Gewebematerial (6) und eine Zwischenschicht aus stark absorbierendem Material (7) zusammengerollt sind, um einen Rohling zu bilden, aus dem Laminatfolien der gewünschten Größe heraus gestanzt werden, **dadurch gekennzeichnet, dass** ein Walzdruck angewendet wird, der dem Rohling eine solche Steifigkeit verleiht, dass die Laminatfolien, die aus ihm herausgestanzt werden, selbsttragend sind und am Unterteil eines Sammelbeutels (1; 11) platziert werden können, der zum Aufnehmen von Urin oder anderen organischen Körperflüssigkeiten gedacht ist, und wobei der Walzdruck das Gewebematerial in den Flächenschichten der Laminatfolien zusammenpresst, um die Porengröße dieser auf ein solches Maß zu verringern, dass Flüssigkeit, die in kurzem Kontakt mit einer Flächenschicht einer nicht horizontalen Laminatfolie steht, außen an Letzterer herabfließt, ohne in nennenswertem Maße die Flächenschicht zu durchdringen, wobei abgegebene Flüssigkeit, die eine Flüssigkeitssammlung bildet, die in Kontakt mit einer Flächenschicht der Laminatfolie steht, Letztere durchdringt, so dass sie von dem stark absorbierenden Material absorbiert wird, das sich innen an der Flächenschicht befindet, und so dass dieses stark absorbierende Material in eine Gelzusammensetzung (14) umgewandelt wird.

12. Verfahren nach Anspruch 11, **dadurch gekennzeichnet, dass** das Gewebematerial in den Flächenschichten der Laminatfolien auf eine Dichte von 0,7 g / cm³ ± 20 %, vorzugsweise 0,7 g / cm 3 ± 10 %, gepresst wird.

13. Verfahren nach Anspruch 11 oder 12, **dadurch gekennzeichnet, dass** das Gewebematerial in solchem Maße gepresst wird, dass es eine Porengröße von 200 µm ± 100 µm, vorzugsweise 250 µm ± 50 µm annimmt.

14. Verfahren nach einem der Ansprüche 11 - 13, **dadurch gekennzeichnet, dass** mindestens die Seitenkanten der ausgestanzten Laminatfolien befeuchtet sind, so dass das stark absorbierende Material, das sich zwischen den Kantenabschnitten der Flächenschichten befindet, in eine Gelzusammensetzung (10) umgewandelt wird, die die Seitenkanten der Laminatfolien schließt.

15. Verfahren nach Anspruch 14, **dadurch gekennzeichnet, dass** die Seitenkanten mithilfe von Wasserdampf befeuchtet sind.

16. Verfahren nach einem der Ansprüche 11 bis 15, **dadurch gekennzeichnet, dass** das stark absorbierende Material in der Laminatfolie von dem unteren Endabschnitt der Laminatfolie aufwärts in gegenseitig getrennte Abschnitte (8) geteilt ist.

17. Verfahren nach Anspruch 16, **dadurch gekennzeichnet, dass** die Teilung der Laminatschicht durch die Flächenschichten erfolgt, die entlang quer verlaufender Verbindungslinien (9) gefügt und verbunden werden.

## Revendications

1. Laminé (5) pour l'agencement dans une poche d'un dispositif pour recueillir de l'urine ou d'autres fluides corporels organiques, comprenant un matériau super absorbant (7) qui est enfermé entre deux couches de surface perméables aux liquides (6) et qui forme une composition de gel (14) quand du fluide délivré est absorbé, **caractérisé en ce que** les couches de surface (6) du laminé (5) présentent une taille de pore spécifiquement adaptée de sorte que du fluide délivré en bref contact avec une couche de surface du laminé (5) non horizontal coulera sur l'extérieur de ce dernier sans pénétrer de manière sensible à travers la couche de surface (6), tandis que du fluide délivré qui forme une accumulation de fluide en contact avec une couche de surface du laminé pénètre à travers ce dernier de façon à être absorbé par le matériau super absorbant (7) situé à l'intérieur de la couche de surface et de façon à transformer ce matériau super absorbant (7) en une composition de gel (14).

2. Laminé selon la revendication 1, **caractérisé en ce qu'**il contient 30 g ± 5 g, de préférence 28 g ± 3 g de matériau super absorbant (7).

3. Dispositif pour recueillir de l'urine ou d'autres fluides corporels organiques, comprenant une poche souple (1 ; 11) fabriquée en un matériau de film imperméable aux liquides, un élément (2 ; 12) pour recevoir du fluide et le transférer dans la poche, un moyen (13) pour empêcher du fluide de quitter la poche par le biais dudit élément de réception, et au moins un corps d'absorption agencé dans la poche (1), **caractérisé en ce que** ledit au moins un corps d'absorption est un laminé (5) selon la revendication 1 ou 2.

4. Dispositif selon la revendication 3, **caractérisé en ce que** le laminé (5) est supporté par le fond de la poche (1), **en ce que** les couches de surface (6) du laminé sont fabriquées en un matériau souple qui permet au laminé de s'affaisser vers le fond de la poche lorsque le matériau super absorbant (7) dans le laminé est transformé en une composition de gel (14) par le fluide délivré.

5. Dispositif selon la revendication 3 ou 4, **caractérisé en ce que** les couches de surface (6) du laminé (1) sont fabriquées en un matériau textile comprimé.

6. Dispositif selon la revendication 5, **caractérisé en ce que** le matériau textile dans les couches de surface (6) est comprimé pour obtenir une densité de 0,7 g / cm³ ± 20 %, de préférence 0,7 g / cm³ ± 10 %.

7. Dispositif selon l'une quelconque des revendications 5 - 6, **caractérisé en ce que** le matériau textile constitue 2 - 6 pour cent en poids, de préférence environ 3 pour cent en poids du laminé.

8. Dispositif selon l'une quelconque des revendications 5 - 7, **caractérisé en ce que** le matériau textile présente une taille de pore de 200 µm ± 100 µm, de préférence de 250 µm ± 50 µm.

9. Dispositif selon l'une quelconque des revendications 3 - 8, **caractérisé en ce que** les portions de bord du laminé (5) sont fermées avec un fil de gel (10) consistant en un matériau super absorbant (7) transformé en gel et agencé entre les portions de bord des couches de surfaces (6).

10. Dispositif selon l'une quelconque des revendications 3 - 9, **caractérisé en ce que** le laminé (5) est divisé en sections (8) à partir de la portion d'extrémité inférieure du laminé et vers le haut, **en ce que** chaque section (8) contient une partie du matériau super absorbant (7) compris dans le laminé, **en ce que** le matériau dans une section est maintenu séparé du matériau dans des sections adjacentes de sorte que du fluide délivré dans la poche (1 ; 11) transforme le matériau super absorbant (7), dans le laminé (5) supporté par le fond de la poche, en une composition de gel (14) une section après l'autre, en commençant dans la section située au fond de la poche.

11. Procédé pour produire des laminés super absorbants (5) selon la revendication 1 ou 2, dans lequel deux couches de matériau textile (6) et une couche intermédiaire de matériau super absorbant (7) sont laminées ensemble pour former un flan à partir duquel des laminés de taille souhaitée sont estampés, **caractérisé en ce qu'**une pression de laminage est utilisée qui donne au flan une telle rigidité que les laminés estampés à partir de celui-ci sont autoportants et peuvent être placés dans le fond d'une poche de collecte (1 ; 11) destinée à recevoir de l'urine ou d'autres fluides corporels organiques, et laquelle pression de laminage comprime le matériau textile dans les couches de surface des laminés afin de réduire la taille de pore de ceux-ci à un tel niveau que du fluide en bref contact avec une couche de surface d'un laminé non horizontal coulera sur l'extérieur de ce dernier sans pénétrer de manière sensible à travers la couche de surface, tandis que du fluide délivré qui forme une accumulation de fluide en contact avec une couche de surface du laminé pénètre à travers ce dernier de façon à être absorbé par le matériau super absorbant situé sur l'intérieur de la couche de surface et de façon à transformer ce matériau super absorbant en une composition de gel (14).

12. Procédé selon la revendication 11, **caractérisé en ce que** le matériau textile dans les couches de surface des laminés est comprimé pour obtenir une densité de 0,7 g / cm³ ± 20 %, de préférence 0,7 g / cm³ ± 10 %.

13. Procédé selon la revendication 11 ou 12, **caractérisé en ce que** le matériau textile est comprimé à un tel niveau qu'il obtient une taille de pore de 200 µm ± 100 µm, de préférence de 250 µm ± 50 µm.

14. Procédé selon l'une quelconque des revendications 11 - 13, **caractérisé en ce qu'**au moins les bords latéraux des laminés estampés sont humidifiés de sorte que le matériau super absorbant situé entre les portions de bord des couches de surface est transformé en une composition de gel (10) fermant les bords latéraux des laminés.

15. Procédé selon la revendication 14, **caractérisé en ce que** lesdits bords latéraux sont humidifiés à l'aide de vapeur d'eau.

16. Procédé selon l'une quelconque des revendications 11 - 15, **caractérisé en ce que** le matériau super absorbant dans le laminé est divisé en sections séparées les unes des autres (8), à partir de la portion d'extrémité inférieure du laminé vers le haut.

17. Procédé selon la revendication 16, **caractérisé en ce que** la division du laminé est effectuée **en ce que** les couches de surface sont jointes et collées les unes aux autres le long de lignes de connexion transversales (9).
